# FASCICULE DE BREVET EUROPEEN

(11) **EP 2 671 543 B1**
(45) Date de publication et mention de la délivrance du brevet: **05.08.2020**
(21) Numéro de dépôt: 13171046.9
(22) Date de dépôt: 07.06.2013
(51) Int. Cl.: A61F 2/38, A61F 2/30

(54) **Composant fémoral de prothèse de genou, méthode de fabrication d'un tel composant et prothèse comprenant un tel composant**
Femoralkomponente einer Knieprothese, Herstellungsmethode einer solchen Komponente, und eine solche Komponente umfassende Prothese
Femoral component of a knee prosthesis, method for manufacturing such a component and prosthesis comprising such a component

(30) Priorité: 08.06.2012 FR 1255377
(43) Date de publication de la demande: 11.12.2013
(73) Titulaire: Corin Limited, The Corinium Centre Cirencester GL7 1YJ (GB)
(72) Inventeur: Faure, Eric, 38190 LAVAL (FR); Goubet, Nicolas, 38190 BERNIN (FR); Forti, Maurizio, 10120 TORINO (IT); Courage, Olivier, 76620 LE HAVRE (FR); Leonardi, Francesco, 12038 SAVIGLIANO (IT); Darman, Zulgael, 33000 BORDEAUX (FR); Vieira da Silva, Manuel, 4715-261 Braga (PT)
(74) Mandataire: Hocking, Adrian Niall

(56) Documents cités:
- EP-A1- 2 436 341
- WO-A1-99/58167
- US-A1- 2002 138 150
- US-A1- 2007 150 067
- US-A1- 2011 125 202
- US-A1- 2011 230 973

## Description

L'invention a trait à un composant fémoral de prothèse de genou, tel qu'utilisé dans une prothèse totale ou partielle du genou.

Dans le domaine des prothèses en général, et tout particulièrement dans le domaine des prothèses de genou, divers techniques sont utilisés pour immobiliser un composant prothétique sur un os. On peut utiliser des vis de fixation ou un ciment orthopédique, ces matériels nécessitant des étapes de poses spécifiques et pouvant induire un descellement ou des réactions allergiques dans le cas du ciment. Il est également connu d'utiliser une fixation dite « biologique » dans laquelle un revêtement est appliqué sur les parties d'un composant prothétique destinées à être en appui contre une partie reséquée d'un os pour favoriser la repousse osseuse et assurer un ancrage pérenne du composant prothétique. Les revêtements utilisés à cette fin sont, par exemple, des plasmas spray Titane revêtus ou non d'Hydroxyapatite, des revêtements par frittage de poudre, de billes ou de fibres.

Il est ainsi connu de FR A 2 934 770 de revêtir la face interne d'un composant fémoral de prothèse de genou d'un revêtement de phosphate de calcium. Cette approche est compatible avec l'utilisation de plots d'ancrage tronconique, mais pas d'une cage intercondylienne.

Il est par ailleurs connu de US A 2007/015 0067 d'utiliser des structures de surface différentes sur différentes parties d'un composant prothétique fémoral, afin d'obtenir des propriétés d'adhérence distinctes, notamment pour recevoir un ciment ou faciliter la repousse osseuse. Ainsi, toutes les surfaces internes du composant fémoral sont sensées être fermement attachées à l'os.

D'autre part, WO-A-99/58167 enseigne d'équiper un composant fémoral de prothèse de genou d'une surface de fixation qui s'étend, entre autres, sur une cage intercondylienne.

Enfin, EP-A-2 486 341 enseigne d'utiliser des régions poreuses dans des surfaces d'un composant fémoral de prothèse d'épaule destinées à interagir avec l'os. D'autre part, des cavités de réception d'un ciment sont prévues sur des surfaces de fixation.

Or, il est parfois nécessaire de procéder au retrait d'un composant fémoral de prothèse de genou, notamment dans des cas de révisions pour descellement, malposition ou douleur chez le patient. Un ostéotome est parfois utilisé pour couper les ponts d'os qui résultent de la croissance osseuse à l'interface entre le fémur et les surfaces du composant revêtues du revêtement favorisant la repousse osseuse. Dans le cas où le composant est équipé d'une cage intercondylienne, c'est-à-dire d'une partie destinée à être engagée entre les deux condyles du fémur, cette cage intercondylienne n'est pas directement accessible depuis l'extérieur lorsque le composant fémoral est en place sur l'extrémité inférieure (distale) du fémur. Il n'est donc pas possible d'agir avec un ostéotome pour couper l'os sur les surfaces internes de la cage intercondylienne, c'est-à-dire les surfaces tournées vers le fémur. Dans ce cas, le composant fémoral de prothèse doit être retiré en force, avec un risque d'arrachement d'un volume important d'os autour de la cage intercondylienne.

C'est à ces inconvénients qu'entend plus particulièrement remédier l'invention en proposant un nouveau composant fémoral de prothèse de genou dont l'ancrage sur l'extrémité inférieure d'un fémur est pérenne et qui peut, en cas de besoin, être retiré sans générer de traumatisme osseux important.

A cet effet, l'invention concerne un composant fémoral de prothèse de genou comprenant une portion externe convexe ainsi qu'une cage intercondylienne qui comprend une surface interne supérieure et deux surfaces internes latérales, ce composant étant revêtu, sur au moins une surface interne destinée à être en appui contre une partie reséquée d'un fémur, d'une couche de revêtement favorisant la repousse osseuse. Conformément à l'invention, cette couche de revêtement est apposée exclusivement sur des surfaces internes du composant qui ne font pas partie de la cage intercondylienne.

Grâce à l'invention, la repousse osseuse n'est pas favorisée sur la cage intercondylienne et la liaison intime entre l'os et le composant fémoral est limitée à des surfaces accessibles à un ostéotome en cas de retrait du composant fémoral.

Selon des aspects avantageux mais non obligatoires de l'invention, un tel composant peut incorporer une ou plusieurs des caractéristiques suivantes prises dans toute combinaison techniquement admissible :
- La couche de revêtement s'étend continûment sur au moins deux surfaces planes internes contigües du composant qui ne sont pas coplanaires.
- La couche de revêtement est d'un seul tenant.
- La couche de revêtement comprend deux bandes latérales disposées de part et d'autre de la cage intercondylienne et qui s'étendent selon une direction antéro-postérieure du composant.
- La couche de revêtement comprend au moins une portion de jonction entre les bandes latérales.
- Cette portion de jonction s'étend sur une partie postérieure du composant, en arrière de la cage intercondylienne.
- La couche de revêtement comprend une portion qui prolonge une première bande latérale en direction de la deuxième bande latérale, en contournant la cage intercondylienne et en rejoignant ou non la deuxième bande latérale.
- La portion qui prolonge la première bande latérale s'étend, par rapport à la cage intercondylienne, à l'opposé de la portion de jonction.

L'invention concerne également une méthode de fabrication d'un composant fémoral de prothèse tel que mentionné ci-dessus et, plus précisément, une méthode de fabrication d'un composant fémoral de prothèse de genou, ce composant comprenant une portion convexe destinée à être en appui contre un plateau tibial, ainsi qu'une cage intercondylienne. Conformément à l'invention, cette méthode comprend également au moins une étape consistant à appliquer, sur au moins une surface interne du composant destinée à être en appui contre une partie reséquée d'un fémur et à l'exclusion d'une surface interne de la cage intercondylienne, une couche de revêtement favorisant la repousse osseuse.

En outre, l'invention concerne une prothèse de genou comprenant un composant fémoral tel que mentionné ci-dessus ou fabriqué avec la méthode mentionnée ci dessus.

Enfin, l'invention concerne un procédé de retrait, à l'aide d'un outil coupant, tel qu'un ostéotome, d'un composant fémoral tel que mentionné ci dessus dans lequel on utilise l'outil coupant pour sectionner la repousse osseuse uniquement en regard des surfaces revêtues de la couche de revêtement favorisant la réponse osseuse, sans agir avec cet outil au niveau des surfaces internes de la cage intercondylienne.

L'invention sera mieux comprise et d'autres avantages de celle-ci apparaîtront plus clairement à la lumière de la description qui va suivre d'un mode de réalisation d'un composant fémoral de prothèse de genou conforme en son principe et d'une méthode de fabrication de ce composant, donnée uniquement à titre d'exemple et faite en référence aux dessins annexés dans lesquels :
- la figure 1 est une vue en perspective d'un composant fémoral conforme à l'invention,
- les figures 2 à 4 sont des vues en perspective analogues à la figure 1, selon d'autres angles et
- la figure 5 et une vue de dessus du composant des figures 1 à 4.

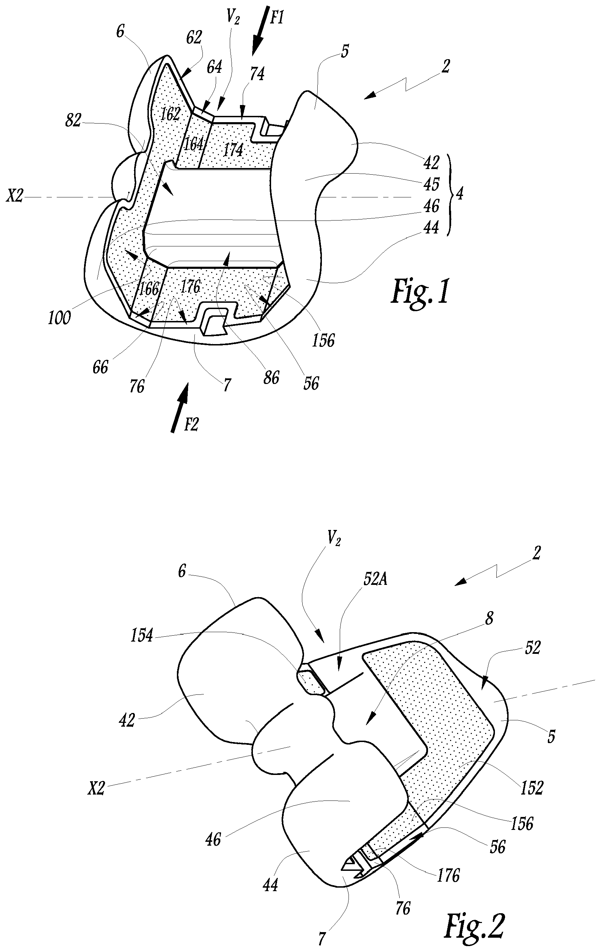

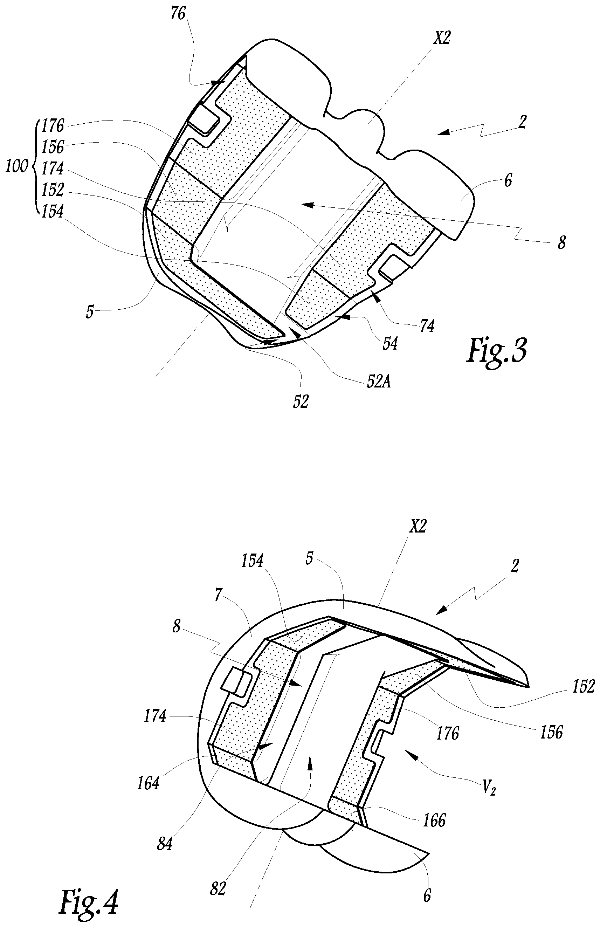

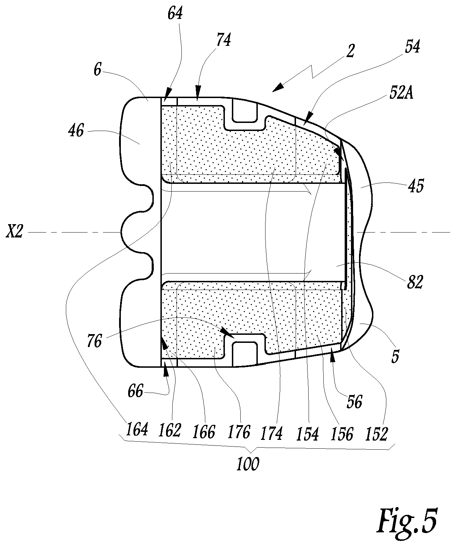

Le composant prothétique fémoral 2 visible aux figures 1 à 5 est réalisé en alliage métallique, par exemple en alliage à base de Chrome-Cobalt ou Titane ou en céramique, par exemple à base de céramique d'alumine.

Dans ce qui suit, le composant est décrit dans une position montée lorsque le patient se tient debout. Ainsi, une partie inférieure ou distale du composant est orientée vers le tibia et une direction avant-arrière ou antéro-postérieure est perpendiculaire à l'axe de l'articulation du genou et sensiblement horizontale.

Ce composant comprend une portion externe convexe 4 qui définit deux condyles 42 et 44 prévus pour venir en appui sur un insert en polyéthylène fixé sur un composant tibial d'une prothèse de genou.

On note X2 un axe longitudinal du composant 2 qui s'étend selon une direction antéro-postérieure, entre une cloison avant 5 et une cloison arrière 6. La surface externe 45, respectivement 46, d'une cloison 5 ou 6 constitue une surface avant ou antérieure, respectivement arrière ou postérieure, de la portion externe 4.

Le composant 2 défini un volume V₂ de réception de l'extrémité inférieure ou distale du fémur d'un patient, après résection de cette extrémité. Ce volume est délimité à l'avant par la cloison 5, à l'arrière par la cloison 6 et vers le bas par un fond 7 qui est globalement parallèle à l'axe X2 et relie les cloisons 5 et 6.

Ainsi, en vu de côté, le composant 2 présente une forme générale en U à fond interne plat.

Le composant 2 comprend également une cage intercondylienne 8 qui est destiné à s'insérer dans le fémur du patient, entre les condyles naturels. Cette cage 8 s'étend globalement selon l'axe X2, en surélévation par rapport au fond 7. Elle a, en section dans un plan perpendiculaire à l'axe X2, une forme globalement rectangulaire à coins tronqués.

On considère les surfaces de la cage intercondylienne 8 orientées vers le volume V2 comme des surfaces internes de cette cage. Cette cage comprend donc une surface interne supérieure 82 parallèle aux surfaces internes du fond 7 et deux surfaces internes latérales gauche 84 et droite 86. Des chanfreins relient les surfaces 82 et 84, d'une part, 82 et 86, d'autre part.

On note respectivement 74 et 76 les surfaces internes supérieures du fond 7 qui sont disposées à gauche et à droite de la cage 8. Ces surfaces sont parallèles à la surface 82.

On note 52 la surface interne de la cloison 5, c'est-à-dire la surface de cette cloison tournée vers la cloison 6. On note 62 la surface interne de la cloison 6, c'est-à-dire la surface de cette cloison tournée vers la cloison 5.

Les surfaces 52, 62, 74 et 76 sont planes. La surface 62 est perpendiculaire à l'axe X2. Les surfaces 52 et 62 divergent ou s'éloignent du fond 7, avec un angle de divergence égal à 5,5°. En pratique, cet angle est compris entre 1 et 10°, de préférence entre 5 et 6°. Le caractère quasi parallèle et divergeant des surfaces 52 et 62 assure un effet de « mise en pression » de l'extrémité réséquée du fémur lors de la pose du composant 2.

On note 54 une surface interne de la cloison 5 qui est plane et relie les surfaces 74 et 52. On note 56 une surface interne de la cloison 5 qui est plane et relie les surfaces 76 et 52. De la même façon, on note 64 et 66 des surfaces internes de la cloison 6 qui relient les surfaces 74 et 76 à la surface 62. Les surfaces 54, 56, 64 et 66 sont inclinées à environ 45° par rapport à l'axe X2.

Conformément à l'invention, une couche 100 d'un matériau favorisant la repousse osseuse est déposée sur les surfaces 52, 54, 56, 62, 64, 66, 74 et 76 sans recouvrir les surfaces 82, 84 et 86.

En d'autres termes, le revêtement favorisant la repousse osseuse est déposé sur des surfaces internes du composant 2 qui sont accessibles par les côtés gauche et droit du composant, dans le sens des flèches F1 et F2 à la figure 1, c'est-à-dire perpendiculairement à l'axe X2, lorsque le composant 2 est immobilisé sur l'extrémité reséquée du fémur d'un patient.

Le produit utilisé comme revêtement favorisant la repousse osseuse peut être un plasma spray Titane revêtu ou non d'Hydroxyapatite, un revêtement obtenu par frittage de poudre, de billes ou de fibres.

La couche 100 de matériau favorisant la repousse osseuse comprend deux bandes latérales 174 et 176 respectivement apposées sur les surfaces 74 et 76 et qui s'étendent de part et d'autre de la cage intercondylienne 8 parallèlement à l'axe X2, c'est-à-dire selon une direction avant/arrière.

La couche 100 comprend également deux bandes 164 et 166 apposées sur les surfaces 64 et 66 ainsi qu'une bande 162 apposée sur la surface 62. Les bandes 162, 164 et 166 constituent ensemble une portion de la couche 100 qui relie les bandes 174 et 176.

Par ailleurs, une bande 154 de la couche 100 est apposée sur la surface 54, alors qu'une autre bande 156 de la couche 100 est apposée sur la surface 56. Enfin, une bande 152 de la couche 100 est apposée sur la surface 52. La bande 156 relie ensemble les bandes 176 et 152. Ainsi, les bandes 156 et 152 constituent ensemble une portion de la couche 100 qui prolonge la bande 176 en direction de la bande 174, sur l'intérieur de la cloison avant 5.

On relève que les bandes 152 et 154 ne sont pas jointives, de sorte qu'il demeure une partie 52A de la surface 52 qui n'est pas revêtue de la couche 100. En variante, les bandes 152 et 154 pourraient être jointives et la surface 52 serait alors presque complètement revêtue de la couche 100. La couche 100 serait alors en forme de boucle fermée entourant la cage 8

La couche 100, qui est constituée des bandes 152, 154, 156, 162, 164, 166, 174 et 176 est d'un seul tenant. Elle est gauche puisqu'elle s'étend sur des surfaces internes du composant 2 qui sont contigües sans être coplanaires.

Lors de la pose du composant 2, après résection de l'extrémité inférieure du fémur, le composant est mis en place en engageant la cage intercondylienne 8 dans le dégagement ménagé à cet effet dans le fémur, les condyles 42 et 44 venant approximativement à la place des condyles naturels.

S'il s'avère ultérieurement nécessaire de retirer le composant 2, il est possible d'agir avec un ostéotome ou tout autre outil coupant dans le sens des flèches F1 et F2 sur la figure 1, pour sectionner l'os qui a repoussé, en coupant les ponts osseux entre la prothèse et l'os, en faisant longer à l'ostéotome ou à l'outil coupant les surfaces 52, 54, 56, 62, 64, 66, 74 et 76.

Il n'est pas nécessaire d'utiliser l'ostéotome au niveau des surfaces 82, 84 et 86 puisque celles-ci ne sont pas revêtues de la couche 100, de sorte que la repousse osseuse au niveau de ses surfaces est peu marquée.

Selon une variante non représentée de l'invention, on peut prévoir d'apposer sur les surfaces 82, 84 et 86 un revêtement empêchant la repousse osseuse, ou plus précisément un revêtement empêchant l'accrochage de l'os sur ses surfaces. En variante, l'état de surface de ses surfaces 82, 84 et 86 peut être d'un type qui ne facilite pas cet accrochage, par exemple de type « poli miroir ».

## Revendications

1. Composant fémoral (2) de prothèse de genou comprenant une portion externe convexe (4) et une cage intercondylienne (8) qui comprend une surface interne supérieure (82) parallèle aux surfaces internes du fond du composant et deux surfaces internes latérales (84), ledit composant ayant une forme générale en U à fond interne plat et étant revêtu, sur au moins une surface interne (52, 54, 56, 62, 64, 66, 74, 76) destinée à être en appui contre une partie reséquée d'un fémur, d'une couche (100) de revêtement favorisant la repousse osseuse, **caractérisé en ce que** la surface interne supérieure (82) est fermée et **en ce que** la couche (100) de revêtement est apposée exclusivement sur des surfaces internes (52, 54, 56, 62, 64, 66, 74, 76) du composant (2) qui ne font pas partie de la cage intercondylienne (8).

2. Composant selon la revendication 1, **caractérisé en ce que** la couche (100) de revêtement s'étend continûment sur au moins deux surfaces planes internes (52, 54, 56, 62, 64, 66, 74, 76) contigües du composant qui ne sont pas coplanaires.

3. Composant selon la revendication 2, **caractérisé en ce que** la couche (100) de revêtement est d'un seul tenant.

4. Composant selon l'une des revendications précédentes, **caractérisé en ce que** la couche (100) de revêtement comprend deux bandes latérales (174, 176) disposées de part et d'autre de la cage intercondylienne (8) et qui s'étendent selon une direction antero-postérieure du composant.

5. Composant selon la revendication 4, **caractérisé en ce que** la couche (100) de revêtement comprend au moins une portion (162, 164, 166) de jonction entre les bandes latérales (174, 176).

6. Composant selon la revendication 5, **caractérisé en ce que** la portion de jonction (162, 164, 166) s'étend sur une partie postérieure (6) du composant, en arrière de la cage intercondylienne (8).

7. Composant selon l'une des revendications 4 à 6, **caractérisé en ce que** la couche (100) de revêtement comprend un portion (152, 156) qui prolonge une première bande latérale (176) en direction de la deuxième bande latérale (174), en contournant la cage intercondylienne (8) et en rejoignant ou non la deuxième bande latérale.

8. Composant selon les revendications 4 et 7, **caractérisé en ce que** la portion (152, 156) qui prolonge la première bande latérale (176) s'étend, par rapport à la cage intercondylienne (8), à l'opposé de la portion de jonction (162, 164, 166).

9. Méthode de fabrication d'un composant fémoral (2) de prothèse de genou, ce composant ayant une forme générale en U à fond interne plat et comprenant une portion convexe (4) et une cage intercondylienne (8) comprenant une surface interne supérieure (82) fermée, ladite méthode comprenant au moins une étape consistant à appliquer, sur au moins une surface interne (52, 54, 56, 62, 64, 66, 74, 76) parallèle aux surfaces internes du fond du composant destinée à être en appui contre une partie reséquée d'un fémur et à l'exclusion d'une surface interne (82, 84, 86) de la cage intercondylienne (8), une couche (100) de revêtement favorisant la repousse osseuse.

10. Prothèse de genou comprenant un composant fémoral (2) selon l'une des revendications 1 à 8 ou fabriqué avec la méthode de la revendication 9.

## Patentansprüche

1. Femorale Komponente (2) einer Knieprothese, umfassend einen konvexen Außenabschnitt (4) und einen interkondylären Käfig (8), der eine obere Innenfläche (82) parallel zu den Innenflächen des Bodens der Komponente und zwei seitliche Innenflächen (84) umfasst, wobei die Komponente eine allgemeine U-Form mit einem flachen inneren Boden aufweist und auf mindestens einer zur Unterstützung eines resezierten Teils eines Femurs vorgesehenen Innenfläche (52, 54, 56, 62, 64, 66, 74, 76) mit einer Überzugsschicht (100) beschichtet ist, die das Knochenwachstum fördert, **dadurch gekennzeichnet, dass** die obere Innenfläche (82) geschlossen ist und die Überzugsschicht (100) ausschließlich auf Innenflächen (52, 54, 56, 62, 64, 66, 74, 76) der Komponente (2) angebracht ist, die nicht Teil des interkondylären Käfigs (8) sind.

2. Komponente nach Anspruch 1, **dadurch gekennzeichnet, dass** sich die Überzugsschicht (100) kontinuierlich über mindestens zwei aneinandergrenzende flache Innenflächen (52, 54, 56, 62, 64, 66, 74, 76) der Komponente erstreckt, die nicht koplanar sind.

3. Komponente nach Anspruch 2, **dadurch gekennzeichnet, dass** die Überzugsschicht (100) einteilig ist.

4. Komponente nach einem der vorhergehenden Ansprüche, **dadurch gekennzeichnet, dass** die Überzugsschicht (100) zwei Seitenstreifen (174, 176) umfasst, die auf beiden Seiten des interkondylären Käfigs (8) angeordnet sind, und die sich in einer antero-posterioren Richtung der Komponente erstrecken.

5. Komponente nach Anspruch 4, **dadurch gekennzeichnet, dass** die Überzugsschicht (100) mindestens einen Verbindungsabschnitt (162, 164, 166) zwischen den Seitenstreifen (174, 176) umfasst.

6. Komponente nach Anspruch 5, **dadurch gekennzeichnet, dass** sich der Verbindungsabschnitt (162, 164, 166) über einen hinteren Teil (6) der Komponente hinter dem interkondylären Käfig (8) erstreckt.

7. Komponente nach einem der Ansprüche 4 bis 6, **dadurch gekennzeichnet, dass** die Überzugsschicht (100) einen Abschnitt (152, 156) umfasst, der einen ersten Seitenstreifen (176) in Richtung des zweiten Seitenstreifens verlängert (174), den interkondylären Käfig (8) umgeht und den zweiten Seitenstreifen erreicht oder nicht erreicht.

8. Komponente nach Anspruch 4 und 7, **dadurch gekennzeichnet, dass** sich der Abschnitt (152, 156), der den ersten Seitenstreifen (176) verlängert, in Bezug auf den interkondylären Käfig (8) gegenüber dem Verbindungsabschnitt (162, 164, 166) erstreckt.

9. Verfahren zur Herstellung einer femoralen Komponente (2) einer Knieprothese, wobei die Komponente eine allgemeine U-Form mit einem flachen inneren Boden aufweist und einen konvexen Abschnitt (4) und einen interkondylären Käfig (8) mit einer geschlossenen oberen Innenfläche (82) umfasst, wobei das Verfahren mindestens eine umfasst Schritt, der darin besteht, auf mindestens einer zur Unterstützung eines resezierten Teils eines Femurs vorgesehenen Innenfläche (52, 54, 56, 62, 64, 66, 74, 76) parallel zu den Innenflächen des Bodens der Komponente und unter Ausschluss einer Innenfläche (82, 84, 86) des interkondylären Käfigs (8), eine Überzugsschicht (100) aufzubringen, die das Knochenwachstum fördert.

10. Knieprothese, umfassend eine femorale Komponente (2) nach einem der Ansprüche 1 bis 8 oder hergestellt nach dem Verfahren nach Anspruch 9.

## Claims

1. Femoral component (2) of a knee prosthesis comprising a convex external portion (4) and an intercondylar cage (8) which comprises an upper internal surface (82) that is parallel to the internal surfaces of the base of the component and two lateral internal surfaces (84), said component having a generally U-shaped design with a flat internal base and coated, on at least one internal surface (52, 54, 56, 62, 64, 66, 74, 76) intended to rest against a resected part of a femur, by a coating layer (100) promoting bone regrowth, **characterized in that** the upper internal surface (82) is closed and that the coating layer (100) is exclusively affixed on internal surfaces (52, 54, 56, 62, 64, 66, 74, 76) of the component (2) which are not part of the intercondylar cage (8).

2. Component according to Claim 1, **characterized in that** the coating layer (100) extends continuously over at least two contiguous planar internal surfaces (52, 54, 56, 62, 64, 66, 74, 76) of the component which are not coplanar.

3. Component according to Claim 2, **characterized in that** the coating layer (100) is a single unit.

4. Component according to any one of the previous claims, **characterized in that** the coating layer (100) comprises two lateral bands (174, 176) laid out on the one side and the other side of the intercondylar cage (8) and which extend along an anterior-posterior direction of the component.

5. Component according to Claim 4, **characterized in that** the coating layer (100) comprises at least one joining portion (162, 164, 166) between the lateral bands (174, 176).

6. Component according to Claim 5, **characterized in that** the joining portion (162, 164, 166) extends along a posterior part (6) of the component, behind the intercondylar cage (8).

7. Component according to any one of Claims 4 through 6, **characterized in that** the coating layer (100) comprises a portion (152, 156) which lengthens a first lateral band (176) in the direction of the second lateral band (174), by going around the intercondylar cage (8), and reaching, or not, the second lateral band.

8. Component according to the Claims 4 and 7, **characterized in that** the portion (152, 156) which lengthens the first lateral band (176) extends, in relationship to the intercondylar cage (8), opposite to the joining portion (162, 164, 166).

9. Method of manufacturing a femoral component (2) of a knee prosthesis, said component having a generally U-shaped design with a flat internal base and comprising a convex portion (4) and an intercondylar cage (8) comprising a closed upper internal surface (82), said method comprising at least one step consisting in the application of a coating layer (100) promoting bone regrowth, on at least one internal surface (52, 54, 56, 62, 64, 66, 74, 76) that is parallel to the internal surfaces of the base of the component intended to rest against a resected part of a femur and with the exclusion of an internal surface (82, 84, 86) of the intercondylar cage (8).

10. Knee prosthesis comprising a femoral component (2) according to any one of Claims 1 through 8 or manufactured with the method of Claim 9.
